# EUROPÄISCHE PATENTSCHRIFT

(11) **EP 3 075 405 B1**
(45) Veröffentlichungstag und Bekanntmachung des Hinweises auf die Patenterteilung: **27.03.2019**
(21) Anmeldenummer: 16169459.1
(22) Anmeldetag: 11.09.2014
(51) Int. Cl.: A61M 15/08, A61M 15/00, B65D 50/04, B05B 11/00, A61M 11/00

(54) **AUSTRAGVORRICHTUNG**
DISCHARGE DEVICE
DISPOSITIF DE DISTRIBUTION

(30) Priorität: 10.10.2013 DE 102013220492
(43) Veröffentlichungstag der Anmeldung: 05.10.2016
(62) Teilanmeldung aus: 14762016.5
(73) Patentinhaber: Aptar Radolfzell GmbH, 78315 Radolfzell (DE)
(72) Erfinder: Greiner-Perth, Jürgen, 78244 Gottmadingen (DE); Krampen, Gerald, 78315 Radolfzell (DE); Szymiczek, Christoph, 78224 Singen (DE)
(74) Vertreter: Patentanwaltskanzlei Cartagena

(56) Entgegenhaltungen:
- EP-A2- 0 799 646
- DE-A1- 10 051 753
- DE-U1- 9 312 423
- GB-A- 689 225
- US-A- 2 586 502
- US-A- 4 099 639
- US-A- 4 801 093

## Beschreibung

### Anwendungsgebiet und Stand der Technik

Die Erfindung betrifft eine Austragvorrichtung für einen Spender zum Austrag von pharmazeutischen Flüssigkeiten umfassend ein Gehäuse mit einer Kopplungsgeometrie zur Anbringung an einem Flüssigkeitsspeicher und eine Nasenolive mit einer an einem distalen Ende angeordneten Austragöffnung. Die Erfindung betrifft weiter einen Spender zum Austrag von pharmazeutischen Flüssigkeiten umfassend einen Flüssigkeitsspeicher und eine Austragvorrichtung.

Die mit gattungsgemäßen Austragvorrichtungen und Spendern auszutragenden Flüssigkeiten umfassen auch Flüssigkeiten, deren Einnahme schädlich sein kann, wenn sie nicht medizinisch indiziert ist. Insbesondere problematisch ist es, wenn Kinder beispielsweise aus Neugierde oder beim Spielen pharmazeutische Spender verwenden und dabei Flüssigkeit austragen und einnehmen. Je nach Flüssigkeit kann dies mit erheblichen Gefahren für die Gesundheit eines Kindes einhergehen.

Die gegebene Problemsituation hat in vielen Ländern der Welt zu gesetzgeberischen Maßnahmen geführt, denen zufolge bei bestimmten pharmazeutischen Flüssigkeiten oder Wirkstoffen nur Spender Verwendung finden dürfen, die üblicherweise durch Kinder nicht geöffnet oder betätigt werden können, da Kinder die Funktionsweise der Inbetriebnahme nicht durchschauen.

Die Handhabung entsprechender Spender oder Austragvorrichtungen mit einer Kindersicherung ist vielfach mühsam. Insbesondere ältere Menschen treffen daher häufig Vorkehrungen, um die Kindersicherung zu umgehen. Dies bringt dann wieder die Gefahr mit sich, dass Kindern ein ungehinderter Zugriff auf die in den manipulierten Spendern bevorrateten Medien möglich wird.

Eine gattungsgemäße Austragvorrichtung ist aus der US 4 801 093 A bekannt.

### Aufgabe und Lösung

Aufgabe der Erfindung ist es, gattungsgemäße Austragvorrichtungen und Spender vorteilhaft weiterzubilden.

Diese Aufgabe wird durch eine Auftragvorrichtung gemäß Anspruch 1 gelöst.

Bei der erfindungsgemäßen Austragvorrichtung ist an einer Außenmantelfläche der Nasenolive ein Außengewinde zur Anbringung einer mit Innengewinde versehenen Schutzkappe vorgesehen. Dieses Außengewinde unmittelbar an der Nasenolive kann mit einem Innengewinde an der Schutzkappe zusammenwirken.

Durch mindestens einen Sperrzahn an der Schutzkappe und mindestens ein Gegenzahn an der Applikatorspitze wirken diese derart zusammen, dass bei einer Verdrehung der Schutzkappe relativ zu der Applikatorspitze in eine erste Richtung zum Aufschrauben der Schutzkappe der mindestens eine Sperrzahn den mindestens einen Gegenzahn überläuft und nach Überlaufen der mindestens eine Sperrzahn und der mindestens eine Gegenzahn eine Verdrehung in die der ersten Richtung entgegengesetzte Richtung zum Abschrauben der Schutzkappe verhindern. Die Schutzkappe und die Applikatorspitze können dann derart aufeinander abgestimmt sein, dass nach Überlaufen des mindestens einen Gegenzahns die Austragöffnung in Kontakt mit einem Deckelbereich der Schutzkappe steht und die Austragöffnung durch die Schutzkappe abgedichtet verschließbar ist, und die Schutzkappe zumindest abschnittsweise elastisch verformbar ist, um durch elastische Verformung der Schutzkappe den mindestens einen Sperrzahn und den mindestens einen Gegenzahn außer Eingriff zu bringen.

Zum Abnehmen der Schutzkappe sind dabei gleichzeitig seitliche Verformungskräfte und ein Drehmoment aufzubringen. Derartige Mechanismen werden auch als Zusammendrück-Dreh-Verschluss (Engl. "Squeeze and turn closure") bezeichnet. Studien haben gezeigt, dass eine derartige Kraft- oder Bewegungskombination für Kinder schwer, für Erwachsene dagegen intuitiv möglich ist.

Das an der Nasenolive vorgesehene Außengewinde erstreckt sich in einer Ausgestaltung über eine gesamte Länge der Nasenolive. In anderen Ausgestaltungen sind die Gewinde derart gestaltet, dass eine Drehbewegung beim Aufschrauben über einen Winkel kleiner 360°, vorzugsweise kleiner 180° erfolgt. Dadurch wird sichergestellt, dass die Drehbewegung bis zum Überlaufen des Gegenzahns und damit bis Erreichen der gesicherten Endlage durchgeführt wird, und der Nutzer nicht frühzeitig den Vorgang aufgrund von Ermüdung abbricht. Die Gewindeabschnitte sind dabei in vorteilhaften Ausgestaltungen so angeordnet, dass ein die Austragöffnung umgebender Bereich gewindefrei ist. Dadurch ist eine Verwendung ohne Komforteinschränkungen gewährleistet.

Beim Aufschrauben der Schutzkappe überläuft der mindestens eine Sperrzahn der Schutzkappe den mindestens einen Gegenzahn der Applikatorspitze. Nach Überlaufen verhindern der mindestens eine Sperrzahn und mindestens einen Gegenzahn eine Bewegung in eine entgegengesetzte Richtung. Die Zähne sind vorzugsweise derart gestaltet, dass ein Überlaufen mit geringen Kräften möglich ist, einer Bewegung in entgegengesetzte Richtungjedoch höhere Kräfte entgegenstehen.

Die Schutzkappe ist zumindest abschnittsweise elastisch verformbar. Die Schutzkappe ist zu diesem Zweck in vorteilhaften Ausgestaltungen aus einem elastisch verformbaren Kunststoff, beispielsweise aus Polypropylen gefertigt. Die Verformung wird dabei sowohl beim Überlaufen als auch beim Lösen des Eingriffs genutzt. In einer Ausgestaltung ist vorgesehen, dass der Sperrzahn der Schutzkappe selbst elastisch verformt wird. Alternativ oder zusätzlich wird in anderen Ausgestaltungen ein den Sperrzahn tragender Bereich verformt. Am Ende der Bewegung zum Überlaufen des mindestens einen Gegenzahns springt die Schutzkappe und/oder der Sperrzahn in die ursprüngliche Form zurück. Ein Umspringen ist vorzugsweise mit einem Knackgeräusch verbunden, welches dem Nutzer das Erreichen der gesicherten Endlage signalisiert.

In vorteilhaften Ausgestaltungen ist die Schutzkappe einteilig. Als einteilig wird im Sinne der vorliegenden Erfindung auch eine ggf. aus mehreren Einzelteilen, z. B. durch Schrauben, Kleben, Schweißen oder jegliche andere Art einer Verbindung fest zusammengefügte Schutzkappe verstanden. Vorzugsweise ist die Schutzkappe als Ein- oder Mehr-Komponenten-Spritzgussteil gefertigt.

Die Schutzkappe kann auf die Applikatorspitze derart abgestimmt sein, dass die Schutzkappe ausreichend weit auf die Applikatorspitze aufschraubbar ist, um die Austragöffnung durch die Schutzkappe abgedichtet zu verschließen. In einer Ausgestaltung weist die Schutzkappe an einem Deckelbereich ein Dichtelement auf, wobei die Schutzkappe über das Dichtelement in Kontakt mit der Austragöffnung ist. In vorteilhaften Ausgestaltungen wird ein an der Austragöffnung anliegender Deckelbereich der Schutzkappe beim Aufschrauben und Überlaufen des Gegenzahns elastisch verformt. Der Deckelbereich liegt dabei unter Wirkung der elastischen Rückstellkräfte an der Applikatorspitze an. In vorteilhaften Gestaltungen liegt eine innenseitige Fläche der Schutzkappe im aufgeschraubten Zustand an der Austragöffnung an und verschließt diese hierdurch. Für eine erhöhte Anpresskraft weist der Deckelbereich in einer Ausgestaltung eine Wölbung in Richtung Innenraum der Schutzkappe auf. In anderen Ausgestaltungen weist die Schutzkappe im Bereich einen Fortsatz auf, der beim Aufschrauben der Schutzkappe in die Austragöffnung einrückt.

In einer Ausgestaltung sind an der an der Applikatorspitze zwei diametral angeordnete Gegenzähne und an der Schutzkappe zwei damit zusammenwirkende, diametral angeordnete Sperrzähne vorgesehen. Dadurch ist eine sichere Verriegelung möglich. Für eine Verformung der Schutzkappe durch einen Nutzer zum Entfernen der Schutzkappe weist diese dabei in vorteilhaften Ausgestaltungen zwei zwischen den Sperrzähnen angeordnete Kraftangriffsflächen auf. Die Kraftangriffsflächen weisen in einer Ausgestaltung eine Oberflächengestaltung auf, welche ein einfaches Ergreifen zum Aufbringen der notwendigen Verformungs- und Drehkräfte erlaubt.

In einer Ausgestaltung ist vorgesehen, dass der mindestens eine Sperrzahn, vorzugsweise zwei diametral angeordnete Sperrzähne, von einem dem Gehäuse zugewandten freien Ende abragt/abragen und in eine Ausnehmung einführbar ist bzw. zwei komplementäre Ausnehmungen sind, an welcher (jeweils) ein Gegenzahn vorgesehen ist. In anderen Ausgestaltungen ist vorgesehen, dass der mindestens eine Gegenzahn, vorzugsweise zwei diametral angeordnete Gegenzähne, von einer der Schutzkappe zugewandten Fläche abragt/abragen und in eine Ausnehmung einführbar ist bzw. zwei komplementäre Ausnehmungen sind, an welcher (jeweils) ein Sperrzahn vorgesehen ist. In einer vorteilhaften Ausgestaltung ist vorgesehen, dass der mindestens eine Sperrzahn und der mindestens eine Gegenzahn jeweils in Radialrichtung mit entgegengesetzter Orientierung abragen, Vorzugsweise ragt der mindestens eine Sperrzahn von einer inneren Mantelfläche der Schutzkappe nach innen ab und wirkt mit einem radial nach außen ragendem Gegenzahn zusammen.

In einer weiteren Ausgestaltung ist an der Applikatorspitze mindestens ein Anschlag vorgesehen, mittels welchem die Verdrehung der Schutzkappe relativ zu der Applikatorspitze in die erste Richtung zum Aufschrauben der Schutzkappe nach Überlaufen des mindestens einen Gegenzahns begrenzt ist. Für den Nutzer ist dabei das Erreichen der gesicherten Endlage haptisch aufgrund der erhöhten Widerstandskraft erfassbar. In vorteilhaften Ausgestaltungen erreicht der mindestens eine Sperrzahn der Schutzkappe den Anschlag bevor ein freies Ende der Schutzkappe auf einer Gegenfläche der Austragvorrichtung anliegt. Dadurch ist auch bei großen Toleranzen sichergestellt, dass die gesicherte Endlage beim Aufschrauben erreicht wird und der Schraubvorgang nicht aufgrund eines Verkantens der Bauteile oder dergleichen abgebrochen wird. Vorzugsweise ist der mindestens eine Sperrzahn der Schutzkappe asymmetrisch gestaltet und weist eine mit dem Gegenzahn zusammenwirkende erste Flanke und eine in Umfangsrichtung dazu beabstandete, mit dem Anschlag der Applikatorspitze zusammenwirkende zweite Flanke auf. Die zweite Flanke ist vorzugsweise kürzer gestaltet, als die erste Flanke.

In einer vorteilhaften Ausgestaltung weist die Schutzkappe zwei koaxial ausgerichtete Hülsenabschnitte auf, wobei das Innengewinde an einem inneren Hülsenabschnitt und der mindestens eine Sperrzahn an einem äußeren Hülsenabschnitt angeordnet sind. Der äußere Hülsenabschnitt ist dabei derart gestaltbar, dass eine Verformung durch Aufbringen einer geeignet gewählten Kraft für eine ergonomische Handhabung und eine kindersichere Anbringung möglich ist. Durch die Anbringung des Innengewindes an einem getrennten Hülsenabschnitt ist eine gleichzeitige Verformung des Innengewindes beim Aufbringen der Kraft verhindert oder auf ein für die Handhabung akzeptables Maß reduziert.

In einer Weiterbildung ist vorgesehen, dass der mindestens eine Sperrzahn an einem dem Deckelbereich gegenüberliegenden Ende des äußeren Hülsenabschnitts und das Innengewinde an einem dem Deckelbereich gegenüberliegenden Ende des inneren Hülsenabschnitts angeordnet sind. Eine Befestigung an der Applikatorspitze erfolgt somit in einem von der Austragöffnung entfernten Bereich.

In einer vorteilhaften Ausgestaltung weist die Schutzkappe an einer äußeren Mantelfläche eine optisch und/oder haptisch erfassbare Markierung auf, welche eine Handhabung zum Aufschrauben und/oder Abschrauben der Schutzkappe symbolisch und/oder als Text beschreibt. Dabei hat sich gezeigt, dass Kinder in der Regel auch symbolische Darstellungen notwendiger Handhabungsschritte zum Abnehmen der Schutzkappe nicht deuten können. Erwachsene können diese dagegen in der Regel sprachunabhängig einfach erfassen. Die Markierung erfolgt in einer Ausgestaltung farblich, wobei ein Aufdruck oder dergleichen angebracht ist. In anderen Ausgestaltungen ist eine Markierung mittels eines Mehr-Komponenten-Spritzguss realisiert.

In vorteilhaften Ausgestaltungen erfolgt eine Markierung mittels Relief. In einer Ausgestaltung ist die äußere Mantelfläche elliptisch zusammenlaufend gestaltet, wobei die reliefartige Markierung auf einer Oberfläche mit einer geraden kreiszylindrischen Hüllkurve vorgesehen ist. Dadurch ist ein ästhetisch ansprechendes und fertigungstechnisch optimiertes Relief geschaffen. Die Markierung liegt auf einer "eigenen" Oberfläche, welche in Radialrichtung betrachtet teilweise hinter und teilweise vor der äußeren Mantelfläche liegt. Dadurch ergibt sich ohne zusätzliche farbliche Gestaltung eine gute optische Erfassbarkeit aufgrund einer Licht-/ Schattenwirkung.

Der Flüssigkeitsspeicher ist in einer Ausgestaltung als sogenannte Quetschflasche gestaltet, wobei durch Aufbringen einer seitlichen Verformungskraft ein Austrag der bevorrateten Flüssigkeit erzielt wird.

Erfindungsgemäß ist vorgesehen, dass die Austragvorrichtung eine gegenüber dem Gehäuse in einer Axialrichtung verlagerbare Betätigungseinheit mit Fingerauflagefläche umfasst, wobei mittels der Betätigungseinheit aus dem Flüssigkeitsspeicher stammende Flüssigkeit durch die Austragöffnung in eine umgebende Atmosphäre abgegeben werden kann. Bei einer besonders bevorzugten Ausgestaltung der Austragvorrichtung ist vorgesehen, dass diese über eine Kolbenpumpe mit einem Pumpenzylinder und einem Pumpenkolben verfügt, die gemeinsam eine Pumpenkammer begrenzen und die durch Kraftbeaufschlagung der Betätigungseinheit zum Zwecke des Flüssigkeitsaustrages bei gleichzeitiger Verringerung eines Volumens der Pumpkammer aus einer Ausgangsrelativlage bis in eine Endrelativlage verlagerbar sind. Dabei ist in einer Ausgestaltung ein Rückströmkanal vorgesehen, der nach Überschreiten einer Zwischenrelativlage die Pumpkammer mit dem Flüssigkeitsspeicher verbindet. Bei einer derartigen Ausgestaltung wird die Betätigung der Betätigungseinheit mittels der Schutzkappe nicht verhindert. Aufgrund der abdichtend aufgesetzten Schutzkappe kommt es jedoch nicht zu einem Austrag der Flüssigkeit. In einer anderen Ausgestaltung hat die Kolbenpumpe keinen Rück- oder Überströmkanal. Die Flüssigkeit in der Pumpkammer kann somit nicht in den Flüssigkeitsspeicher entweichen. Die in der Pumpkammer vorhandene Flüssigkeit steht daher einer Bewegung Betätigungseinheit zum Zwecke des Flüssigkeitsaustrages entgegen. Die Schutzkappe sperrt dabei zwar nicht mechanisch, jedoch aufgrund der Abdichtung der Austrittsöffnung hydraulisch eine Bewegung der Betätigungseinheit. Vorzugsweise ist dabei die Nasenolive ortsfest mit der Betätigungseinheit verbunden. Bei einer derartigen Austragvorrichtung ist somit eine Austragöffnung vorgesehen, welche gemeinsam mit der Betätigungseinheit gegenüber dem Gehäuse verlagerbar ist.

In einer Ausgestaltung ist die Betätigungseinheit drehbar und Axialrichtung verlagerbar in dem Gehäuse gelagert. Um eine Gegenkraft zum Auf- oder Abschraubend er Schutzkappe aufzubringen, ergreift der Nutzer dabei mit einer zweiten Hand die Betätigungseinheit. In vorteilhaften Ausgestaltungen ist die Betätigungseinheit dagegen drehfest und in Axialrichtung verlagerbar in dem Gehäuse gelagert. Dadurch ist es möglich, dass der Nutzer das Gehäuse oder die Betätigungseinheit oder - bei drehfester Kopplung mit dem Gehäuse - den Flüssigkeitsspeicher ergreift. Dadurch ist eine besonders ergonomisch günstige Handhabung erreicht.

Die Kraftangriffsflächen der Schutzkappe, an welchen eine seitliche Verformungskraft und ein Drehmoment zum Entfernen der Schutzkappe aufgebracht wird und die Fingerauflagefläche sind vorzugsweise derart gestaltet, dass bei aufgesetzter Schutzkappe die Kraftangriffsflächen mit der Fingerauflagefläche einen Winkel vom ca. 90° einschließen. Dies erlaubt eine gute Kraftaufbringung, wobei eine Hand eines Nutzers ggf. auch an der Fingerauflagefläche geführt werden kann.

Um eine Gegenkraft aufzubringen, weisen in vorteilhaften Ausgestaltungen die Betätigungseinheit und/oder das Gehäuse mindestens eine Grifffläche auf, welche vorzugsweise im Wesentlichen in einer Ebene mit dem mindestens einen an der Applikatorspitze vorgesehenen Gegenzahn liegt. Vorzugsweise sind zwei diametral angeordnete Griffflächen vorgesehen, welche um 90° versetzt zu den Kraftangriffsflächen der Schutzkappe angeordnet sind. Dies erlaubt eine besonders ergonomisch günstige Handhabung. Die Griffflächen schließen vorzugsweise mit einer Ebene der Fingerauflagefläche einen Winkel von ca. 90° ein.

Die Aufgabe wird weiter gelöst durch einen Spender zum Austrag von pharmazeutischen Flüssigkeiten umfassend einen Flüssigkeitsspeicher und eine damit verbundenen Austragvorrichtung mit den oben beschriebenen Merkmalen. Erfindungsgemäße Spender sind insbesondere bei pharmazeutischen Flüssigkeiten zweckmäßig, deren Einnahme mit erheblicher Gefahr einhergeht. Hierzu zählen zum Beispiel Schmerzmittel. Auch eine Verwendung eines erfindungsgemäßen Spenders mit pharmazeutischen Flüssigkeiten / Formulierungen mit nasenschleimhautabschwellenden Wirkstoffen wie beispielsweise Imidazolin ist zweckmäßig.

### Kurzbeschreibung der Zeichnungen

Weitere Aspekte und Vorteile der Erfindung ergeben sich außer aus den Ansprüchen auch aus der nachfolgenden Beschreibung bevorzugter Ausführungsbeispiele der Erfindung, die anhand der Figuren nachfolgend erläutert werden. Dabei zeigen:
- Fig. 1: einen Spender mit einer Austragvorrichtung gemäß einer ersten Ausgestaltung der Erfindung mit aufgesetzter Schutzkappe in einer perspektivischen Darstellung,
- Fig. 2: den Spender gemäß Fig. 1 ohne Schutzkappe in einer perspektivischen Darstellung,
- Fig. 3: die Schutzkappe für den Spender gemäß Fig. 1 in einer Seitenansicht,
- Fig. 4: die Schutzkappe gemäß Fig. 3 in einer geschnittenen Darstellung,
- Fig. 5: den Spender gemäß Fig. 1 in einem Halbschnitt,
- Fig. 6: eine geschnittene Darstellung des Spenders gemäß Fig. 1 entlang einer Schnittebene VI-VI gemäß Fig. 5;
- Fig. 7: eine geschnittene Darstellung des Spenders gemäß Fig. 1 entlang einer Schnittebene VII-VII gemäß Fig. 5;
- Fig. 8: einen Spender mit einer Austragvorrichtung ähnlich Fig. 5 in einem Halbschnitt;
- Fig. 9: ein Detail IX des Spenders gemäß Fig. 8 und
- Fig. 10: einen Spender mit einer Austragvorrichtung in einer perspektivischen Darstellung.

### Detaillierte Beschreibung der Ausführungsbeispiele

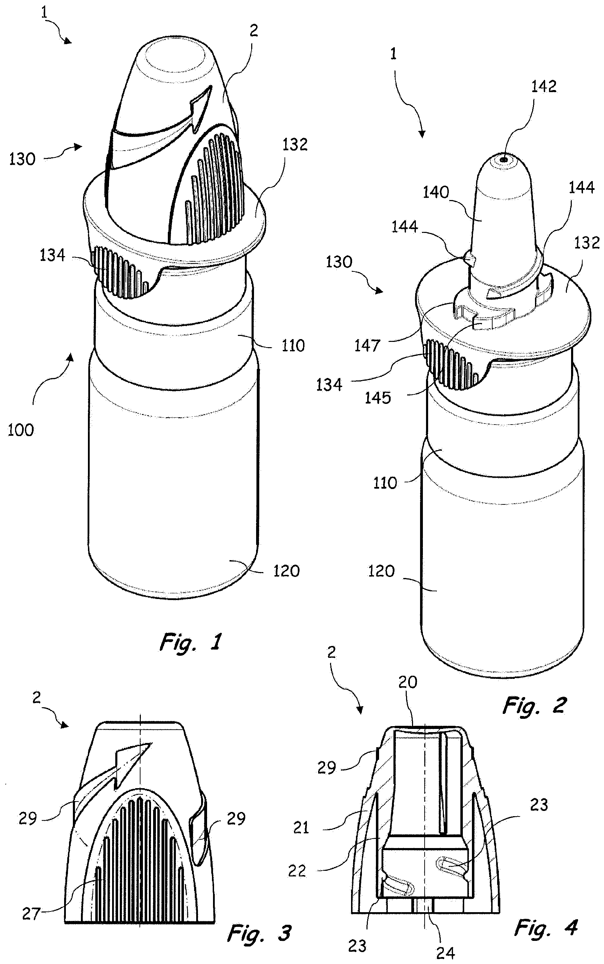

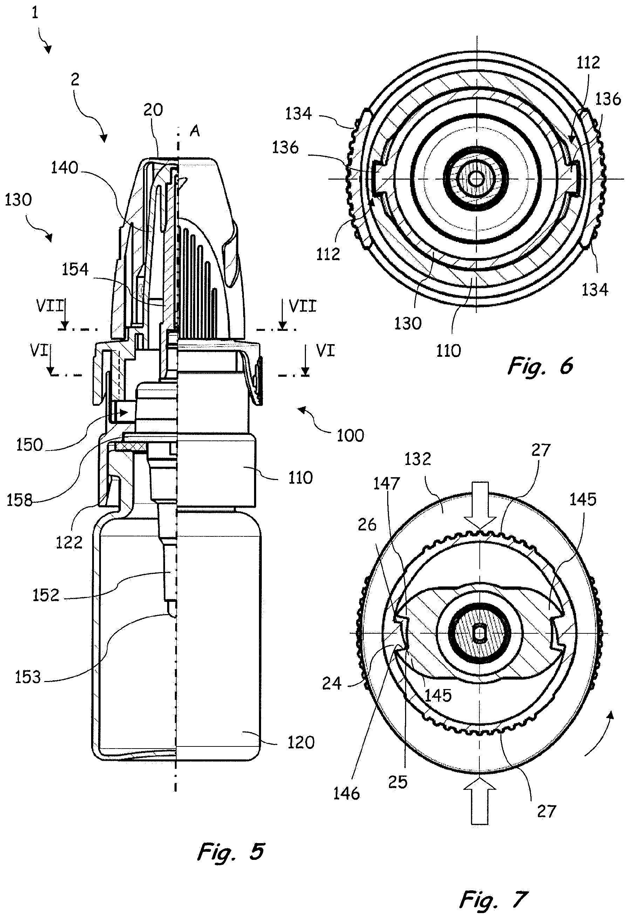

Die Fig. 1 bis 7 zeigen einen Spender 1 mit einer Austragvorrichtung 100 gemäß einer ersten Ausgestaltung der Erfindung. Dabei zeigen Fig. 1 und 2 perspektivische Darstellungen des Nasalspenders 1 mit einer aufgesetzten Schutzkappe 2 bzw. ohne Schutzkappe. Fig. 3 und 4 zeigen die Schutzkappe 2 in einer Seitenansicht und einer Schnittdarstellung. Fig. 5 zeigt den Spender 100 in einem Halbschnitt-Fig. 6 und 7 zeigen geschnittene Draufsichten auf den Spender 100 entlang von Schnittebenen VI-VI bzw. VII-VII gemäß Fig. 5.

Der in den Fig. 1, 2 und 5 bis 7 dargestellte Austragvorrichtung 100 verfügt über ein Gehäuse 110, an welches ein externer Flüssigkeitsspeicher 120 mittels einer Schnappverbindung 122 angekoppelt ist. Die dargestellte Austragvorrichtung 100 weist auf der dem Flüssigkeitsspeicher 120 abgewandten Seite des Gehäuses 110 eine Betätigungseinheit 130 auf, welche über eine auflagefläche 132 verfügt. An dieser Betätigungseinheit 130 ist eine als Nasenolive 140 vorgesehen, an deren distalen Ende eine Austragöffnung 142 angeordnet ist. Die Betätigungseinheit 130 ist gegenüber dem Gehäuse 110 in einer Axialrichtung A verlagerbar, wobei zum Zwecke des Austrags von Flüssigkeit eine Verlagerung der Betätigungseinheit 130 in Richtung auf das Gehäuse 110 zu erforderlich ist.

Innerhalb des Gehäuses 110 ist eine Pumpeinrichtung 150 vorgesehen, deren Einlasskanal 152 in den Flüssigkeitsspeicher 120 hineinragt. Zum Ansaugen von Flüssigkeit ist ein Steigrohr 153 vorgesehen, das in den Figuren verkürzt dargestellt ist. Der Auslasskanal 154 der Pumpeinrichtung 150 ist an der Innenseite der Nasenolive 140 befestigt. Der Hauptkörper 158 der Pumpeinrichtung 150 ist zwischen dem Flüssigkeitsspeicher 120 und dem Gehäuse 110 lagefixiert. Bei einer Verlagerung der Betätigungseinheit 130 mit der Nasenolive 140 in Richtung auf den Flüssigkeitsspeicher 120 zu, wird die Bewegung auf den Auslasskanal 154 der Pumpeinrichtung 150 übertragen, wodurch Flüssigkeit zur Austragöffnung 142 gefördert wird. Dies wird mittels einer internen nicht dargestellten Kolbenpumpe innerhalb der Pumpeinrichtung 150 erzielt. Alternativ könnte auch vorgesehen sein, dass die Flüssigkeit im Flüssigkeitsspeicher 120 druckbeaufschlagt vorliegt und statt der Pumpeinrichtung eine schaltbare Ventileinrichtung den Austrag steuert.

Um einen ungewollten Flüssigkeitsaustrag, beispielsweise durch spielende Kinder, zu verhindern, ist die Schutzkappe 2 vorgesehen, die an der Applikatorspitze 140 befestigt werden kann. Die dargestellte Schutzkappe 2 weist einen Deckelbereich 20 auf, welcher im geschlossenen Zustand wie in Fig. 3 erkennbar an der Austragöffnung 142 anliegt und diese abdichtend verschließt. Die Schutzkappe 2 weist weiter zwei von dem Deckelbereich 20 abragende, koaxial ausgerichtete Hülsenabschnitte 21, 22 auf. In dem dargestellten Ausführungsbeispiel besitzen beide Hülsenabschnitte 21, 22 eine rotationssymmetrische Grundform. Es sind jedoch auch Ausgestaltungen denkbar, bei welchen der äußere Hülsenabschnitt 22 nicht rotationssymmetrisch gestaltet ist.

Die Kopplungsgeometrie zwischen der Schutzkappe 2 und der Applikatorspitze 140 ist als sogenannter "Seitlicher Drück-Dreh-Verschluss" oder "Zusammendrück-Dreh-Verschluss" (Engl. "Squeeze and Turn") gestaltet. Zum Öffnen des Verschlusses und Abnehmen der Schutzkappe 2 ist es notwendig, gleichzeitig an bestimmten Bereichen der Schutzkappe 2 eine Verformungskraft mit Kraftkomponente in Radialrichtung und ein Drehmoment aufzubringen.

Die Schutzkappe 2 und die Nasenolive 140 weisen zu diesem Zweck komplementäre Gewinde auf. In dem dargestellten Ausführungsbeispiel ist an einer Außenmantelfläche der Nasenolive 140 ein Außengewinde 144 umfassend zwei rechtssteigende Gewindeabschnitte vorgesehen. Die Gewindeabschnitte des Außengewindes 144 sind zueinander punktsymmetrisch an der Nasenolive 140 angeordnet und erstrecken sich jeweils über einen Winkel kleiner 180°. An der Schutzkappe 2, genauer an dem inneren Hülsenabschnitt 22, ist ein komplementäres Innengewinde 23 mit zwei Gewindeabschnitten vorgesehen, sodass ein Anbringen der Schutzkappe 2 in zwei um 180° um die Axialrichtung verdrehten Ausrichtungen der Schutzkappe 2 möglich ist.

Die Schutzkappe 2 weist weiter zwei um 180° versetzte Sperrzähne 24 auf, welche mit Gegenzähnen 145 an der Applikatorspitze 140 zusammenwirken, wie am besten in Fig. 7 erkennbar ist. Die Sperrzähne 22 sind an einem dem Deckelbereich 20 gegenüberliegenden Ende des äußeren Hülsenabschnitts 21 angeordnet. In der gegen Abschrauben gesicherten Endlage liegt - wie in Fig. 7 dargestellt - eine Flanke 25 des Sperrzahns 22 an einer Gegenflanke 146 des Gegenzahns 145 an, sodass ein Drehen der der Schutzkappe 2 relativ zu der Applikatorspitze 140 entgegen dem Uhrzeigersinn zum Öffnen des Verschluss und Abnehmen der Schutzkappe 2 gesperrt ist. Durch Aufbringen einer schematisch durch Pfeile angedeuteten Verformungskraft F an zwei, um jeweils 90° zu den Sperrzähnen 24 versetzt angeordneten Kraftangriffsflächen 27 wird die Schutzkappe 2 verformt und der Sperrzahn 24 ist außer Eingriff mit dem Gegenzahn 145 bringbar. Der Nutzer kann zu diesem Zweck die Betätigungseinheit 130 ergonomisch günstig an zwei diametral angeordnete Griffflächen 136 ergreifen. Die Griffflächen 136 sind wenn die Schutzkappe 2 in der gesicherten Endlage wie dargestellt aufgesetzt ist, um 90° zu den Kraftangriffsflächen 27 versetzt. Dies erlaubt eine ergonomisch günstige Kraftaufbringung.

An der Applikatorspitze 140 sind weiterzwei Anschläge 147 vorgesehen, mittels welchen eine Drehung der Schutzkappe 2 relativ zu der Applikatorspitze 140 zum Aufschrauben der Schutzkappe 2 nach Überlaufen der Gegenzähne 145 begrenzt ist. Wie in Fig. 7 erkennbar, sind die zwei Sperrzähne 22 der Schutzkappe 2 jeweils asymmetrisch gestaltet und weisen jeweils die mit dem Gegenzahn 145 zusammenwirkende erste Flanke 25 und eine in Umfangsrichtung dazu beabstandete, mit dem Anschlag 147 zusammenwirkende zweite Flanke 26 auf. Die zweite Flanke 26 ist dabei kürzer als die erste Flanke 25. Die Flanken 25, 26 sind über eine in dem dargestellten Ausführungsbeispiel leicht gewölbte Vorderflanke verbunden. Die Anschläge 147 sind jeweils spiegelsymmetrisch zu den Gegenzähnen 145 gestaltet.

Wie in Fig. 4 erkennbar ist, enden die Gewindeabschnitte des Innengwindes 23 in Umfangsrichtung betrachtet an den Sperrzähnen 22.

Wie ebenfalls in Fig. 4 erkennbar, ist die äußere Mantelfläche des äußeren Hülsenabschnitts 23 elliptisch zusammenlaufend gestaltet. Die äußere Mantelfläche weist eine optisch und/oder haptisch erfassbare Markierung 29 auf, welche eine Handhabung zum Aufschrauben und/oder Abschrauben der Schutzkappe 2 symbolisch beschreibt. Die Markierung 29 ist in dem dargestellten Ausführungsbeispiel als Relief auf einer Oberfläche mit einer geraden kreiszylindrischen Hüllkurve vorgesehen. Die Oberfläche der Markierung 29 steht dabei an einem dem Deckelbereich 20 zugewandten Ende von der äußeren Mantelfläche ab, wohingegen sie an dem gegenüberliegenden Ende eine Vertiefung der Mantelfläche bildet.

Zum Austragen der Flüssigkeit nach Abnehmend er Schutzkappe 2 ist eine Kraft in Axialrichtung A an der Fingerauflagefläche 132 aufzubringen, um die Betätigungseinheit 130 gegenüber dem Gehäuse 110 zu verlagern.

In dem in den Fig. 1 bis 5 dargestellten Ausführungsbeispiel ist die Betätigungseinheit 130 drehfest und in Axialrichtung A verlagerbar in dem Gehäuse 110 gelagert. In dem dargestellten Ausführungsbeispiel ist ein hülsenförmiger Abschnitt der Betätigungseinheit 130 in einem entsprechend als Buchse gestalteten Abschnitt des Gehäuses 110 geführt. Der als Buchse gestaltete Abschnitt des Gehäuse 110 weist für eine drehfeste Führung zwei diametral angeordnete Nuten 112 auf, in welchen zwei an dem hülsenförmiger Abschnitt der Betätigungseinheit 130 vorgesehene Führungsrippen 136 geführt sind.

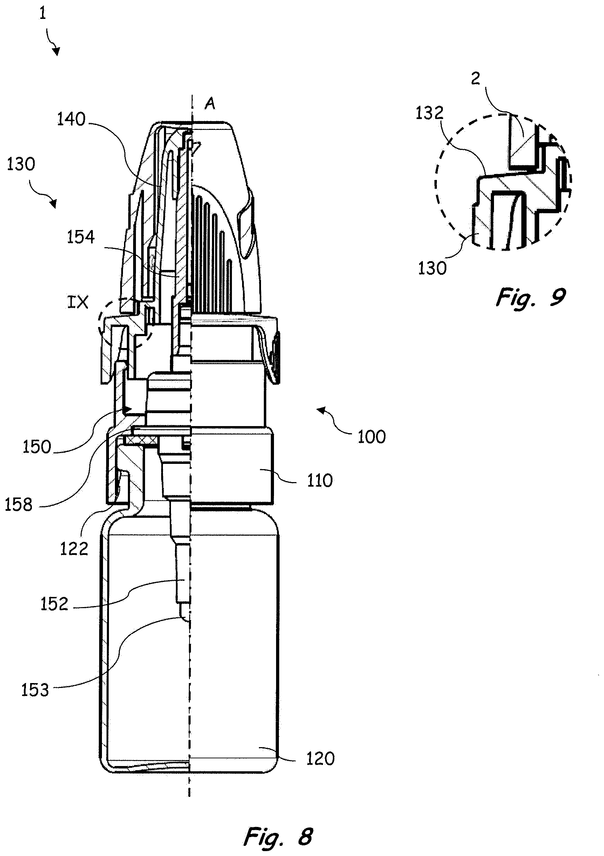

Fig. 8 zeigt eine zweite Ausgestaltung eines Spenders 1 mit einer Austragvorrichtung 100 in einem Halbschnitt. Der Spender 1 und die Austragvorrichtung entsprechen im Wesentlichen dem Spender 1 bzw. der Austragvorrichtungen gemäß den Figuren 1 bis 7. Für gleiche Bauteile werden einheitliche Bezugszeichen verwendet und auf eine erneute Beschreibung wird verzichtet.

Im Unterschied zu dem Spender gemäß den Fig. 1 bis 7 ist die Betätigungseinheit 130 drehbar und in Axialrichtung A verlagerbar in dem Gehäuse 110 gelagert, wobei zu diesem Zweck ebenfalls ein hülsenförmiger Abschnitt der Betätigungseinheit 130 in einem entsprechend als Buchse gestalteten Abschnitt des Gehäuses 110 geführt ist.

Fig. 9 zeigt ein Detail IX gemäß Fig. 8. Wie oben beschrieben, verhindert ein in Fig. 7 dargestellter Anschlag 147 nach Überlaufen des Gegenzahns 145 eine weitere Drehung. Wie in Fig. 9 erkennbar, sind die Bauteile dabei derart aufeinander abgestimmt, dass nach Überlaufen des Gegenzahns 145 ein freies Ende der Schutzkappe 2 noch nicht an der Fingerauflagefläche 132 der Betätigungseinheit 130 anliegt. Eine entsprechende Ausgestaltung ist auch bei der Austragvorrichtung 100 gemäß den Fig. 1 bis 7 vorteilhaft.

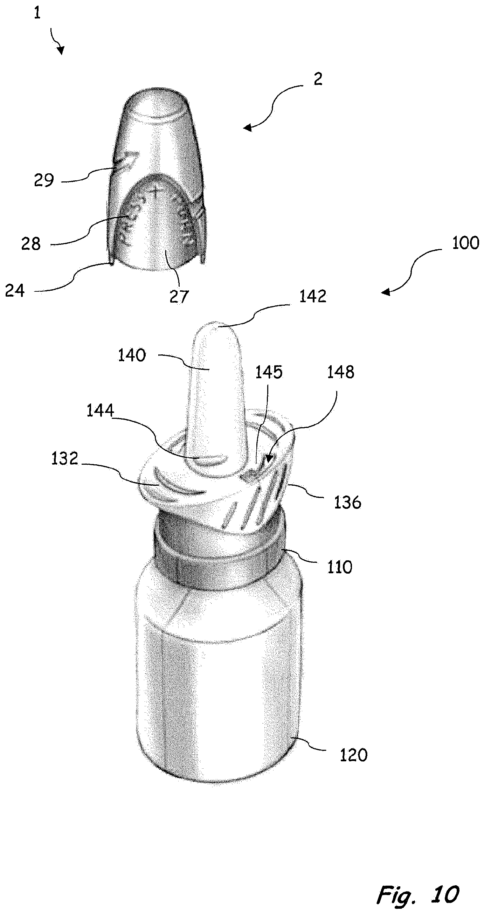

Fig. 10 zeigt schematisch eine weitere Ausgestaltung eines Spenders 1 umfassend eine Austragvorrichtung 100 mit einer angenommen dargestellten Schutzkappe 2 und einen Flüssigkeitsspeicher 120, Auch die Bauteile dieser Ausgestaltung entsprechen im Wesentlichen den Bauteilen gemäß den Figuren 1 bis 7 und für gleiche oder ähnliche Bauteile werden einheitliche Bezugszeichen verwendet.

Die Austragvorrichtung 100 umfasst ein Gehäuse 110 und eine Nasenolive 140 mit einer an einem distalen Ende angeordneten Austragöffnung 142. Zum abdichtenden Verschließen der Austragöffnung dient die Schutzkappe 2. Die Schutzkappe 2 weist ein nicht sichtbares Innengewinde auf, welches mit einem Außengewinde 144 an der Nasenolive 140 zusammenwirkt. Für eine Kindersicherung sind an der Schutzkappe 2 weiter zwei axial abragende Sperrzähne 24 vorgesehen, welche mit ortsfest an der Applikatorspitze 140 angeordneten Gegenzähnen 145 zusammenwirken. Die Gegenzähne 145 sind jeweils in einer Ausnehmung 148 an einer der Schutzkappe 2 zugewandten Fläche vorgesehen. Die Sperrzähne 24 überlaufen unter Verformung der Schutzkappe 2 und/oder der Sperrzähne 24 die Gegenzähne 145 und springen nach einem Überlaufen der Gegenzähne 145 in die Ursprungsform zurück. Die Sperrzähne 24 und die Gegenzähne 145 liegen anschließend aneinander an und verhindern eine Drehbewegung der Schutzkappe 2 relativ zu der Applikatorspitze 140 und damit ein Abnehmen der Schutzkappe 2.

Um die Schutzkappe 2 zu entfernen, ist an zwei zwischen den Sperrzähnen angeordneten Kraftangriffsflächen 27 eine seitliche Verformungskraft aufzubringen. Die notwendige Bewegung ist durch Markierungen 28, 29 an der Schutzkappe 2 angezeigt, Dabei ist sowohl eine Markierung 28 vorgesehen, welche eine Handhabung zum Aufschrauben und/oder Abschrauben der Schutzkappe als Text beschreibt, als auch eine Markierung 29, welche eine Handhabung symbolisch beschreibt,

Die Kraftangriffsflächen 27 sind bei aufgesetzter Schutzkappe 2 um ca. 90° versetzt zu zwei der Applikatorspitze 140 zugeordneten Griffflächen 136 angeordnet. Die Griffflächen 136 stehen senkrecht auf der der Schutzkappe 2 zugewandten Fläche.

Die Applikatorspitze 140 ist in einer Ausgestaltung starr mit dem Gehäuse 110 verbunden. In anderen Ausgestaltungen ist die Applikatorspitze 140 wie oben ortsfest an einer Betätigungseinheit 130 angebracht, wobei die der Schutzkappe 2 zugewandten Fläche als Fingerauflagefläche 132 der Betätigungseinheit dient.

## Patentansprüche

1. Austragvorrichtung für einen Nasalspender zur nasalen Verabreichung eines flüssigen Mediums umfassend
- ein Gehäuse (110) mit einer Kopplungsgeometrie zur Anbringung an einem Flüssigkeitsspeicher (120), und
- eine Applikatorspitze (140) in Form einer Nasenolive (140) mit einer an einem distalen Ende angeordneten Austragöffnung (142),
wobei
an einer Außenmantelfläche der Nasenolive (140) ein Außengewinde (144) zur Anbringung einer mit Innengewinde versehenen Schutzkappe (2) vorgesehen ist und die Austragvorrichtung eine gegenüber dem Gehäuse (110) in einer Axialrichtung (A) verlagerbare Betätigungseinheit (130) mit Fingerauflagefläche (132) umfasst, mit der die Nasenolive (140) ortsfest verbunden ist, wobei mittels der Betätigungseinheit (130) aus dem Flüssigkeitsspeicher (120) stammende Flüssigkeit durch die Austragöffnung (142) in eine umgebende Atmosphäre abgegeben werden kann,
**dadurch gekennzeichnet, dass**
an der Applikatorspitze (140) mindestens ein Gegenzahn (145) oberhalb einer Fingerauflagefläche (132) zur Zusammenwirkung mit einem Sperrzahn an der Schutzkappe (2) vorgesehen ist, wobei dieses Zusammenwirken darin besteht, dass bei einer Verdrehung der Schutzkappe (2) relativ zu der Applikatorspitze (140) in eine erste Richtung zum Aufschrauben der Schutzkappe (2) der mindestens eine Sperrzahn den mindestens einen Gegenzahn (145) überläuft und nach Überlaufen der mindestens eine Sperrzahn und der mindestens eine Gegenzahn (45) eine Verdrehung in die der ersten Richtung entgegengesetzte Richtung zum Abschrauben der Schutzkappe (2) verhindern.

2. Austragvorrichtung nach Anspruch 1,
**dadurch gekennzeichnet, dass**
das Außengewinde (144) sich über die gesamte Länge der Nasenolive (140) vorgesehen ist.

3. Austragvorrichtung nach einem der vorstehenden Ansprüche,
**dadurch gekennzeichnet, dass**
das Außengewinde (144) derart gestaltet ist, dass eine Drehbewegung beim Aufschrauben der Schutzkappe (2) bis in eine Endlage über einen Winkel kleiner 360°, vorzugsweise kleiner 180°, erfolgt.

4. Austragvorrichtung nach einem der Ansprüche 1 oder 3,
**dadurch gekennzeichnet, dass**
Gewindeabschnitte des Außengewindes (144) so angeordnet sind, dass ein die Austragöffnung (142) umgebender Bereich gewindefrei ist.

5. Austragvorrichtung nach einem der vorstehenden Ansprüche,
**dadurch gekennzeichnet, dass**
das Außengewinde (144) zwei rechtssteigende Gewindeabschnitte aufweist, die sich jeweils über einen Winkel kleiner 180° erstrecken.

6. Austragvorrichtung nach einem der vorstehenden Ansprüche,
**dadurch gekennzeichnet, dass**
der Gegenzahn (145) in einer Ausnehmung (148) an der Fingerauflagefläche (132) vorgesehen ist.

7. Austragvorrichtung nach einem der vorstehenden Ansprüche,
**dadurch gekennzeichnet, dass**
die Betätigungseinheit (130) drehfest und in Axialrichtung (A) verlagerbar in dem Gehäuse (110) gelagert ist.

## Claims

1. Discharge device for a nasal dispenser for nasal administration of a liquid medium, comprising
- a housing (110) with a coupling geometry for mounting on a liquid storage unit (120), and
- an applicator tip (140) in the form of a nosepiece (140) with a discharge opening (142) arranged at a distal end,
wherein
an external thread (144) is provided on an outer circumferential face of the nosepiece (140) in order to permit mounting of a protective cap (2) provided with an internal thread, and the discharge device comprises an actuation unit (130) which is displaceable in an axial direction (A) in relation to the housing (110) and has a finger support surface (132) to which the nosepiece (140) is connected in a positionally fixed manner, wherein liquid originating from the liquid storage unit (120) can be dispensed through the discharge opening (142) into a surrounding atmosphere by means of the actuation unit (130),
**characterized in that**
at least one counter-tooth (145) is provided on the applicator tip (140) above a finger support surface (132) and serves to interact with a locking tooth on the protective cap (2), wherein this interaction entails that, during a rotation of the protective cap (2) relative to the applicator tip (140) in a first direction for screwing on the protective cap (2), the at least one locking tooth overruns the at least one counter-tooth (145) and, after it has overrun the latter, the at least one locking tooth and the at least one counter-tooth (45) prevent a rotation in the direction, opposite to the first direction, for unscrewing the protective cap (2).

2. Discharge device according to Claim 1, **characterized in that** the external thread (144) is provided over the entire length of the nosepiece (140).

3. Discharge device according to either of the preceding claims, **characterized in that** the external thread (144) is configured in such a way that a rotational movement, when screwing the protective cap (2) on as far as an end position, takes place over an angle of less than 360°, preferably less than 180°.

4. Discharge device according to either of Claims 1 and 3, **characterized in that** thread portions of the external thread (144) are arranged such that a region surrounding the discharge opening (142) is thread-free.

5. Discharge device according to one of the preceding claims, **characterized in that** the external thread (144) has two right-hand thread portions, which each extend over an angle of less than 180°.

6. Discharge device according to one of the preceding claims, **characterized in that** the counter-tooth (145) is provided in a recess (148) on the finger support surface (132).

7. Discharge device according to one of the preceding claims, **characterized in that** the actuation unit (130) is mounted in a rotationally fixed manner and is displaceable in the housing (110) in axial direction (A).

## Revendications

1. Dispositif de distribution pour un distributeur nasal destiné à l'administration nasale d'un milieu liquide, comprenant:
- un boîtier (110) avec une géométrie de couplage pour le placement sur un réservoir de liquide (120), et
- une pointe d'applicateur (140) sous la forme d'une olive nasale (140) avec un orifice de distribution (142) disposé à une extrémité distale,
dans lequel il est prévu sur une surface latérale extérieure de l'olive nasale (140) un filet extérieur (144) pour le placement d'un capot de protection (2) doté d'un filet intérieur et le dispositif de distribution comprend une unité d'actionnement (130) déplaçable dans une direction axiale (A) par rapport au boîtier (110), avec une face d'application de doigt (132), à laquelle l'olive nasale (140) est assemblée de façon fixe, dans lequel du liquide provenant du réservoir de liquide (120) peut être délivré dans une atmosphère ambiante à travers l'orifice de distribution (142) au moyen de l'unité d'actionnement (130),
**caractérisé en ce qu'**il est prévu sur la pointe d'applicateur (140) au moins une dent opposée (145) au-dessus d'une face d'application de doigt (132) pour coopérer avec une dent de blocage sur le capot de protection (2), dans lequel cette coopération consiste **en ce que** lors d'une rotation du capot de protection (2) par rapport à la pointe d'applicateur (140) dans un premier sens pour visser le capot de protection (2) ladite au moins une dent de blocage franchit ladite au moins une dent opposée (145) et après le franchissement ladite au moins une dent de blocage et ladite au moins une dent opposée (45) empêchent une rotation dans le sens opposé au premier sens pour dévisser le capot de protection (2).

2. Dispositif de distribution selon la revendication 1, **caractérisé en ce que** le filet extérieur (144) est prévu sur toute la longueur de l'olive nasale (140).

3. Dispositif de distribution selon l'une quelconque des revendications précédentes, **caractérisé en ce que** le filet extérieur (144) est configuré de telle manière qu'il se produise un mouvement de rotation lors du vissage du capot de protection (2) jusque dans une position finale sur un angle inférieur à 360°, de préférence inférieur à 180°.

4. Dispositif de distribution selon une des revendications 1 ou 3, **caractérisé en ce que** des parties de filet du filet extérieur (144) sont disposées de telle manière qu'une région entourant l'orifice de distribution (142) soit dépourvue de filet.

5. Dispositif de distribution selon l'une quelconque des revendications précédentes, **caractérisé en ce que** le filet extérieur (144) présente deux parties de filet à droite, s'étendant chacune sur un angle inférieur à 180°.

6. Dispositif de distribution selon l'une quelconque des revendications précédentes, **caractérisé en ce que** la dent opposée (145) est prévue dans un évidement (148) sur la face d'application de doigt (132).

7. Dispositif de distribution selon l'une quelconque des revendications précédentes, **caractérisé en ce que** l'unité d'actionnement (130) est montée de façon calée en rotation et déplaçable en direction axiale (A) dans le boîtier (110).
